# EUROPEAN PATENT SPECIFICATION

(11) **EP 4 098 221 B1**
(45) Date of publication and mention of the grant of the patent: **05.11.2025**
(21) Application number: 21748481.5
(22) Date of filing: 28.01.2021
(51) Int. Cl.: A61C 8/00

(54) **BIOIMPLANT AND METHOD FOR MANUFACTURING BIOIMPLANTS**
BIOIMPLANTAT UND VERFAHREN ZUR HERSTELLUNG VON BIOIMPLANTATEN
BIO-IMPLANT ET PROCÉDÉ DE FABRICATION DE BIO-IMPLANTS

(30) Priority: 31.01.2020 JP 2020014698
(43) Date of publication of application: 07.12.2022
(73) Proprietor: Kyocera Corporation, Kyoto-shi Kyoto 612-8501 (JP)
(72) Inventor: KAMO, Michimasa, Kyoto-shi, Kyoto 612-8501 (JP); ISHIMIZU, Keita, Kyoto-shi, Kyoto 612-8501 (JP); YAMASHITA, Mitsuyoshi, Kyoto-shi, Kyoto 612-8501 (JP); SAWASE, Takashi, Nagasaki-shi, Nagasaki 852-8521 (JP); KUROSHIMA, Shinichiro, Nagasaki-shi, Nagasaki 852-8521 (JP)
(74) Representative: TBK
(86) International application number: PCT/JP2021/002991
(87) International publication number: WO 2021/153658

(56) References cited:
- JP-A- 2011 510 742
- JP-A- H11 511 662
- US-A1- 2009 176 191
- US-A1- 2009 191 507
- US-A1- 2012 316 650
- US-A1- 2015 056 574

## Description

### TECHNICAL FIELD

The present invention relates to a biological implant and a method for manufacturing a biological implant.

### BACKGROUND OF INVENTION

Prior art which is related to this field is disclosed in US2009/176191A1 showing ceramic dental implant, in US 2012/316650A1 showing implants having three distinct surfaces, in US2009/191507A1 showing implant surface with increased hydrophilicity, and in US2015056574 showing process for providing a topography to the surface of a dental implant.
A titanium alloy has a light weight, no magnetism, high corrosion resistance, and high strength, and is thus widely used for orthopedic implants or dental implants. Orthopedic implants include, for example, implants for spine and for joint replacement, and each include a metal body implantable in the bone. The metal body has, on its surface, unevenness at the micrometer to millimeter scale defined with metal particles such as metal beads adhering to the surface. The surfaces of the metal particles are roughened with an etchant at the micrometer to nanometer scale.

### SUMMARY

The present disclosure provides a biological implant according to claim 1.

### BRIEF DESCRIPTION OF DRAWINGS

FIG. 1 is a schematic diagram of an implant illustrating an example structure.
FIG. 2 is a flowchart of an example surface roughening process.
FIG. 3 is a graph showing the relationship between the surface roughness value Ra and the removal torque value.
FIG. 4 is a flowchart of an example method for measuring a surface roughness value.
FIG. 5 is an example captured image of a cut surface of an implant body.
FIG. 6 is an example captured image of a cut surface of an implant body.
FIG. 7 is an example captured image of a surface of an implant body.
FIG. 8 is an example binarized image obtained from a captured image of a cut surface of an implant body.
FIG. 9 is an example captured image of a cut surface of an implant body with a small surface roughness value.
FIG. 10 is an example captured image of a cut surface of an implant body with a large surface roughness value.
FIG. 11 is an example captured image of a porous member.
FIG. 12 is an example high-power-field captured image of the porous member illustrated in FIG. 11 before etching.
FIG. 13 is an example high-power-field captured image of the porous member illustrated in FIG. 11 after etching.
FIG. 14 is a graph showing the relationship between the surface roughness value Rlr and the removal torque value.

### DESCRIPTION OF EMBODIMENTS

A biological implant and the bone are to be secured with a greater force. The present disclosure provides a titanium-alloy biological implant that can be firmly secured to a base such as a biological bone.

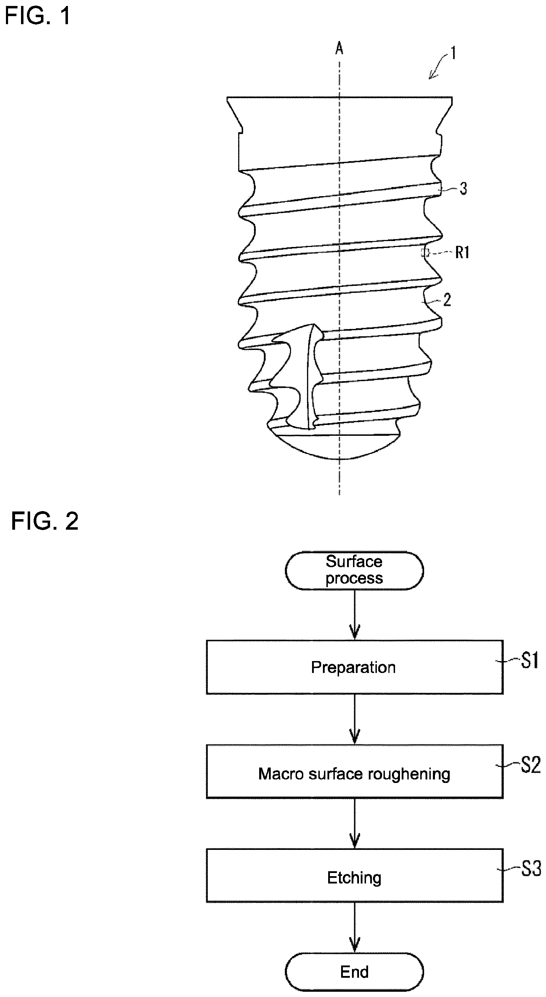

FIG. 1 is a schematic diagram of an implant 1 illustrating an example structure. The implant 1 is, for example, a dental implant, and includes an implant body 2. The implant body 2 is implanted into a part (hereafter referred to as a base) of the living organism such as the bone to secure the implant 1 in the body. The implant body 2 may be continuous and integral with the implant 1, or combined with another component to form the implant 1.

The implant body 2 illustrated in FIG. 1 is substantially cylindrical and elongated in one direction. The implant body 2 includes a threaded portion 3 on its outer circumferential surface. The threaded portion 3 includes a thread protruding outward in the radial direction of the implant body 2 and extending helically from the tip to the head of the implant body 2. The implant body 2 is screwed into the base with a tool (not illustrated).

The implant 1 being a dental implant may be referred to as a fixture.

A dentist removes a deficient portion of a tooth or a defective tooth from a patient's mouth and implants the implant 1 as its replacement.

The implant 1 immediately after being implanted is secured under a frictional resistance force between the threaded portion 3 and the bone. This securing force immediately after the implantation is referred to as an initial securing force. The initial securing force decreases over time with a biological reaction (or bone remodeling). The surface of the implanted implant body 2 is in contact with new bone that forms with a biological reaction. As the new bone gradually increases, the securing force between the implant body 2 and the bone increases.

A therapeutic period with no upper structure such as a tooth crown being attached is typically provided until a sufficiently high securing force is obtained through new bone formation. More specifically, any higher securing force resulting from new bone formation can directly reduce the therapeutic period and the period for which the patient loses occlusion. The implant surface is thus to increase the force of securing the bone through new bone formation.

The inventors have revealed that an increased force of securing the bone between the implant body 2 and a base (e.g., a jawbone) depends on the surface state of the implant body 2 at the sub-micrometer scale. The relationship between the force of securing the bone and the surface state of the implant body 2 at the sub-micrometer scale will now be described in detail.

Although FIG. 1 illustrates the dental implant 1, the implant 1 according to the present embodiment is not limited to a dental implant, and may be applied to, for example, an orthopedic implant.

In a specific example, the implant according to the present embodiment may be applied to an implant for securing the spine. The implant 1 includes multiple implant bodies 2 implantable in multiple spines, attachments at the heads of the implant bodies 2, and a rod coupled to the attachments to connect the multiple implant bodies 2 together. In this case, each implant body 2 is also referred to as a bone screw or a pedicle screw. When each implant body 2 is implanted into the spine and the rod is coupled to the multiple attachments, the multiple spines can be secured together.

The implant 1 may be, for example, an implant for joint replacement. In this case, the implant 1 includes a pair of components forming an artificial joint, and the implant body 2 for securing each component to the bone. The implant body 2 may be shaped as a rod or a curved surface with no threaded portion.

In other words, the implant body 2 in the implant 1 may be of any type implantable in a part (e.g., the bone) of the living organism. For example, the implant 1 may be a cosmetic implant for, for example, aesthetic purposes, in addition to a medical implant.

The implant body 2 is made of a titanium alloy. In this case, the implant body 2 made of a titanium alloy refers to the implant body 2 including a titanium alloy contained in at least the outer circumferential surface. The titanium alloy may be an α alloy, a β alloy, or an α-β alloy. The α alloy is a titanium alloy mainly having an α phase, and the β alloy is a titanium alloy mainly having a β phase. The α-β alloy is a titanium alloy with the α and β phases. The α phase has a crystal structure called a hexagonal close-packed structure. The β phase has a body-centered cubic structure. Examples of the α-β alloy include Ti (titanium)-6Al (aluminum)-4V (vanadium), Ti-6Al-7Nb (niobium), Ti-6Al-2Nb-1Ta (tantalum), Ti-3Al-2.5V, Ti-5Al-2.5Fe (iron), and Ti-5Al-3Mo (molybdenum)-4Zr (zirconium). When Ti-6Al-4V is used as a titanium alloy, the implant body 2 has a light weight, no magnetism, high corrosion resistance, and high strength.

To firmly secure the implant body 2 to the base, the outer circumferential surface of the implant body 2 may undergo surface roughening. For example, the surface undergoes surface roughening (hereafter referred to as macro surface roughening) of the order of several tens of micrometers to millimeters, and then undergoes acid etching.

FIG. 2 is a flowchart of an example surface roughening process performed on an implant body. The implant body 2 before undergoing surface roughening is hereafter referred to as an untreated implant body. **In** the example in FIG. 2, an untreated implant body is prepared first in step S1. The outer circumferential surface of the untreated implant body then undergoes macro surface roughening in step S2. In a specific example of the macro surface roughening, blasting is used. Blasting is a process of blowing a fine abrasive against a process target surface. This increases the surface roughness of the process target surface. Abrasives may be referred to as media. When sand media are used for blasting, the blasting can be particularly referred to as sandblasting.

For example, the untreated implant body is transported into a predetermined blasting machine. The blasting machine blows an abrasive with, for example, the centrifugal force or the wind power against the entire outer circumferential surface of the untreated implant body to cause the abrasive to collide against the outer circumferential surface of the untreated implant body. Examples of the abrasive include at least one of alumina, boron, and borax. Borax is used herein in an example. Hereafter, among workpieces to be the implant body 2, a workpiece that has undergone macro surface roughening is referred to as a semi-treated implant body.

This macro surface roughening can further increase the surface roughness of the outer circumferential surface of a semi-treated implant than the surface roughness of an untreated implant body. In other words, the outer circumferential surface of the semi-treated implant body has unevenness corresponding to the abrasive. More specifically, through the blasting, the outer circumferential surface of the semi-treated implant body has a surface roughness value of greater than or equal to 1 µm and less than or equal to 3 µm. In other words, the blasting conditions (e.g., the size, the speed, and the quantity of the abrasive, and the processing time) are determined to provide a surface roughness value of about 1 to 3 µm. The surface roughness value of the outer circumferential surface of the semi-treated implant body can be measured in accordance with a typical standard, ISO25178. The roughness may be measured in accordance with JIS B0601 and JIS B0633.

In step S3, the outer circumferential surface of the semi-treated implant body undergoes wet etching. Wet etching roughens the outer circumferential surface of the semi-treated implant body through a chemical reaction (or corrosion) between the etchant and the semi-treated implant body by placing the outer circumferential surface of the semi-treated implant body into contact with the etchant.

The semi-treated implant body is transported into a predetermined etching machine. The etching machine includes, for example, a tank to hold the etchant, and the semi-treated implant body is immersed in the etchant. Examples of the etchant include an acid mixture containing an ammonium fluoride, a sulfuric acid, and a hydrogen peroxide solution. After the semi-treated implant body is immersed into the etchant for a predetermined time period (e.g., about several minutes), the outer circumferential surface of the semi-treated implant body is etched, and the implant body 2 can be prepared. This etching defines fine unevenness of the order of several hundreds of nanometers on the outer circumferential surface of the implant body 2.

Although the outer circumferential surface of the implant body 2 has a complex porous structure, the etchant can access the inner surface of the porous structure. Thus, the inner surface of the porous structure can uniformly have unevenness of the order of several hundreds of nanometers.

The above surface roughening can increase the surface roughness of the outer circumferential surface of the implant body 2.

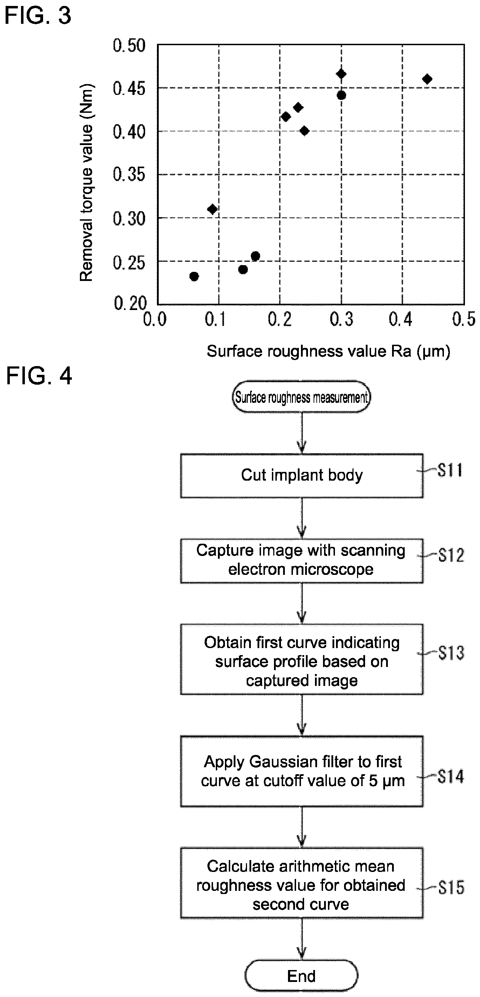

The relationship between the surface roughness value Ra of the outer circumferential surface of the implant body and the securing force on the implant body implanted in the base will now be examined. FIG. 3 is a graph showing the relationship between the surface roughness value Ra and the removal torque value. This relationship is obtained in the experiment described below.

First, multiple implant bodies with different values of surface roughness were prepared. More specifically, six pure-titanium implant bodies and four Ti-6Al-4V implant bodies 2 were prepared. The pure-titanium implant bodies have a shape the same as or similar to that of the implant bodies 2. The pure-titanium implant bodies underwent surface roughening similar to the surface roughening performed on the implant body 2. In this case, the six pure-titanium implant bodies were prepared under varying blasting conditions and etching conditions. Similarly, the four implant bodies 2 were prepared under varying blasting conditions and etching conditions.

More specifically, the pure-titanium implant bodies underwent the processing described below. The abrasives used as appropriate in the blasting process were borax and alumina. The etchants used as appropriate were an oxalic acid and an acid mixture including a hydrochloric acid and a sulfuric acid. The etching time and other conditions were varied as appropriate. A specific implant body underwent etching consecutively using these two etchants to prepare a pure-titanium implant body.

The abrasives used as appropriate in blasting of the Ti-6Al-4V implant bodies 2 were borax and alumina. The etchants used as appropriate were an acid mixture containing a hydrochloric acid and a sulfuric acid, an acid mixture containing a hydrogen fluoride and a nitric acid, and an acid mixture containing an ammonium fluoride, a sulfuric acid, and a hydrogen peroxide solution. A specific implant body did not undergo etching to form the implant body 2.

Each of the prepared pure-titanium implant bodies and the implant bodies 2 was then implanted into the tibia of a Japanese white rabbit (at the age of 20 to 31 weeks, with a weight of 3.5 to 4.0 kg). After implantation, the securing force on each implant body and the bone gradually increases over several weeks through new bone regeneration caused by a biological reaction.

In this case, after four weeks from the implantation of each implant body to the tibia, a torque test was conducted to measure the removal torque value for removing the implant body 2 from the tibia to evaluate the securing force. This torque test was conducted with reference to mechanical testing methods for metallic bone screws JIS T0311. More specifically, the tibia of the rabbit is removed after four weeks from the implantation, the bone piece containing the implant body 2 is secured to a predetermined securing member, and torque is applied in the removal direction to the implant body. The removal direction is a direction in which the implant body is removed from the bone piece. The torque tester includes the securing member, a driving unit, and a torque detector. The securing member secures the bone piece. A tool that applies torque to the implant body is removably attached to the driving unit. The driving unit includes a motor to rotate the tool and apply torque to the implant body. The torque detector detects, for example, the current flowing through the motor, and calculates the torque value based on the current. The driving unit gradually increases the torque applied to the implant body. The torque detector outputs, as a removal torque value, the torque value at the removal of the implant body from the bone piece.

As described above, the removal torque value for each implant body was measured with the torque tester. The number of measured implant bodies is in a range of seven to ten per type (samples that have died during the experiments are excluded).

At the same time, the surface roughness value of each implant body was measured in the manner described below. In the measurement, the implant bodies prepared under the same conditions as those for the implant bodies used for measuring the securing force were used.

The surface treated through macro surface roughening is evaluated with a typical roughness measurement method (e.g., JIS B 0633 and JIS B 0601). The obtained surface roughness value greatly depends on the unevenness resulting from, for example, macro surface roughening observable before acid etching, and thus the unevenness in a sub-micrometer order resulting from acid etching cannot be evaluated as appropriate. Thus, the inventors have established a method of evaluating unevenness in a sub-micrometer order defined through acid etching with a method (hereafter simply referred to as filtering) involving preparing the cross sections of the treated surfaces, obtaining information about unevenness with a scanning electron microscope, and removing the unevenness observable before acid etching through filtering.

FIG. 4 is a flowchart of a method for measuring a surface roughness value. The surface roughness value Ra in FIG. 3 is measured with this measurement method. First, in step S11, an operator cut each implant body along the cut surface orthogonal to the surface of the implant body, for example, along the cut surface including a center axis A (refer to FIG. 1) of the implant body.

In step S12, a scanning electron microscope was used to capture an image of the cut surface of each implant body with, for example, a magnification of 5000. In this case, a scanning electron microscope S-3400N manufactured by HITACHI (registered trademark) was used to capture an image of a part of the threaded portion 3.

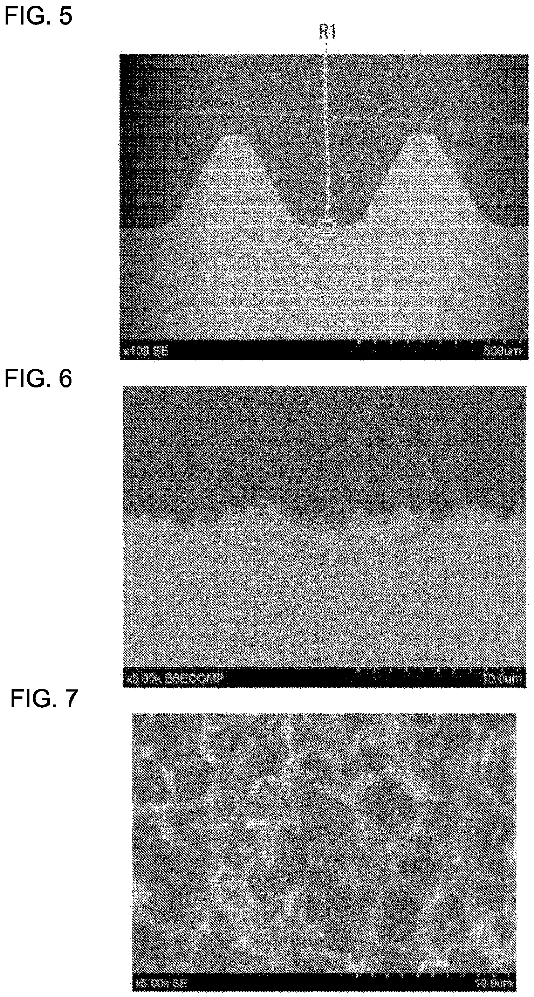

FIGs. 5 and 6 are schematic example captured images. FIG. 5 is a captured image of the cut surface of the implant body 2 captured with a magnification of 100. FIG. 6 is a captured image of the cut surface of the implant body 2 captured with a magnification of 5000.

FIG. 6 is an image including an image capturing area R1 in FIG. 5. In the example in FIG. 5, the image capturing area R1 corresponds to the root of the threaded portion 3 on the outer circumferential surface of the implant body. The image capturing area R1 has a lateral dimension of 25.4 µm and a longitudinal dimension of 19.1 µm.

In each captured image, the pixel value of a pixel group indicating the implant body differs from the pixel value indicating the background. In the examples in FIGs. 5 and 6, the pixel value of the implant body 2 is greater than the pixel value of the background.

FIG. 7 is a captured image of the outer circumferential surface of the implant body 2. The captured image is an image of the surface of the implant body 2 captured with the scanning electron microscope with a magnification of 5000. As seen from FIGs. 6 and 7, the outer circumferential surface of the implant body 2 has fine unevenness.

The captured image in FIG. 6 captured with the scanning electron microscope is input into a predetermined image processing device. The image processing device is an electronic circuit, and includes at least one processor that provides the image processing capacity. The processor includes, for example, at least one circuit or unit to perform at least one data calculation procedure or process by performing an instruction stored in an associated memory. In another embodiment, the processor may be firmware (e.g., a discrete logic component) to perform at least one data calculation procedure or process. All or any of the functions of the image processing device may be implemented by a hardware circuit without using software to implement its functions.

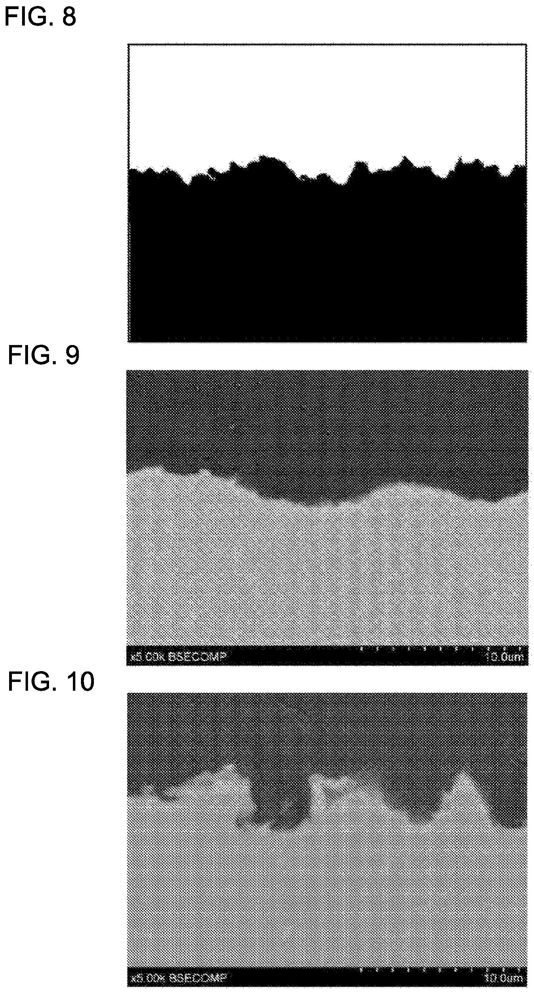

Referring back to FIG. 4, in step S13, the image processing device performs image processing on the captured image, and obtains a first curve indicating the profile of the outer circumferential surface of the implant body 2. In a specific example, the image processing device first binarizes the captured image to obtain a binarized image. FIG. 8 is an example schematic binarized image. The image processing device obtains a first curve from this binarized image. The image processing device performs edge extraction such as a Canny method on the binarized image to obtain an edge image, and performs thinning such as Hilditch thinning on the edge image to obtain the first curve. The first curve is a profile curve of the outer circumferential surface of the implant body 2 in the measurement length of 25.4 µm. Instead of this method, the first curve may be obtained by another algorithm or manual tracing.

In this example, image processing is performed using image processing software WinROOF (trademark) (Mitani Corporation). The image processing device performs the procedures defined by the software to obtain the first curve through the above image processing. The image processing device that runs the software has the function of implementing the operation described below.

The first curve includes a waviness component indicating a surface roughness value of about 1 to 3 µm defined through blasting, and an unevenness component indicating a surface roughness value of the order of several hundreds of nanometers defined through etching. In this example, the waviness component is removed, and the unevenness component is extracted.

Referring back to FIG. 4, in step S14, the image processing device removes the waviness component, or more specifically, a long-wavelength component from the first curve. More specifically, the image processing device applies a Gaussian filter to the first curve at a cutoff value of 5 µm to obtain a second curve. In other words, the image processing device performs high-pass filtering on the first curve using a cutoff value of 5 µm, shorter than the cycle of the surface roughness (waviness component) resulting from blasting, to remove the waviness component resulting from blasting from the first curve and obtain the second curve. Thus, the second curve mainly indicates the surface roughness (unevenness component) defined through etching.

In step S15, the image processing device calculates an arithmetic mean roughness value (hereafter referred to as a surface roughness value Ra) with the second curve. This arithmetic mean roughness value is obtained by averaging the absolute values of the difference between the average value of the second curve in the height direction and each point on the second curve.

FIG. 3 is a graph showing the relationship between the removal torque value and the surface roughness value Ra measured with the above method. In FIG. 3, data for the pure-titanium implant bodies is indicated with rhomboid plotted points, and data for the Ti-6Al-4V implant bodies 2 is indicated with circular plotted points.

As shown in FIG. 3, the removal torque value of the implant bodies with a surface roughness value Ra of less than 0.2 µm is less than or equal to a value of about slightly greater than 0.3 Nm, whereas the removal torque value of the implant bodies with a surface roughness value Ra of greater than or equal to 0.2 µm is about greater than or equal to about 0.4 Nm. As shown in FIG. 3, the removal torque value changes drastically at the surface roughness value Ra of 0.2 µm.

Thus, the implant body 2 may have a surface roughness value Ra of greater than or equal to 0.2 µm. The implant body 2 with this structure can be firmly secured to the base.

FIGs. 9 and 10 are example captured images of cut surfaces of implant bodies with different values of surface roughness Ra. FIG. 9 shows the cut surface of the implant body 2 with a surface roughness value Ra of 0.14 µm. FIG. 10 shows the cut surface of the implant body 2 with a surface roughness value Ra of 0.30 µm. The outer circumferential surface of the implant body 2 in FIG. 9 has gentle unevenness in relatively long cycles. In contrast, the outer circumferential surface of the implant body 2 in FIG. 10 has steep and deep unevenness in relatively short cycles. When the base is the bone, the bone surrounding the implant body 2 grows onto the outer circumferential surface of the implant body 2 over time to fill the unevenness. Thus, the implant body 2 is more firmly secured to the base.

In view of the above, for the implant body 2 having a surface roughness value Ra of 0.2 µm, the drastic rise of the removal torque value around a surface roughness value Ra of 0.2 µm is seemingly caused by the unevenness fully engageable with the base on the outer circumferential surface of the implant body 2.

In contrast, the same acid etching was performed on pure titanium and Ti-6Al-4V samples with a known method. The surface roughness value Ra (defined herein) was measured for these samples. The Ti-6Al-4V sample had a surface roughness value Ra of 0.14 µm. The known methods have not reflected the intended scope of the surface roughness of the sub-micrometer order, nor the difference between the surface states of pure titanium and a Ti alloy obtained by the same acid etching. More specifically, simply using a known manufacturing method for a titanium alloy such as Ti-6Al-4V to prepare a biological implant cannot provide the intended surface roughness. Thus, the implant cannot be firmly secured to the base such as a biological bone.

A known method does not use unevenness in a micrometer level observable before acid etching in evaluating the surface roughness. The implant bodies prepared with known multiple manufacturing conditions each showed a surface roughness value Ra of less than or equal to 0.1 µm. More specifically, when prepared simply with a known manufacturing method, a titanium-alloy biological implant cannot be firmly secured to the base such as a biological bone.

The surface roughness value of typical titanium-alloy dental implants available in the market was measured with the method used herein. None of the Ti-6Al-4V dental implants within the investigated range, although not all are included, had a surface roughness value Ra exceeding 0.2 µm.

More specifically, known titanium-alloy implants had a surface roughness value Ra not exceeding 0.2 µm measured with the method defined herein, revealing that the known titanium-alloy implants cannot be firmly secured to the base such as a biological bone.

The titanium-alloy implant body 2 with a surface roughness value Ra of greater than or equal to 0.2 µm is prepared, for example, under the conditions described below. First, a Ti-6Al-4V untreated implant body is prepared. Blasting (step S2) is then performed on the untreated implant body as macro surface roughening to prepare a semi-treated implant body. Etching (step S3) is then performed on the semi-treated implant body with an acid mixture containing an ammonium fluoride, a hydrogen peroxide solution, and a nitric acid as an etchant. The acid composition is, for example, 20 w/v% of an ammonium fluoride, 6 w/v% of a hydrogen peroxide, and a sulfuric acid (total acidity of 30 w/v%). The processing time is set to be, for example, in a range of ten seconds to ten minutes, 30 seconds to three minutes, or more specifically, about one minute. Thus, a Ti-6Al-4V implant body 2 with a surface roughness value Ra of 0.3 µm was prepared.

In the present embodiment, the titanium-alloy implant body 2 has a surface roughness value Ra of greater than or equal to 0.2 µm. Thus, the implant body 2 can be implanted into and secured to the base with a sufficiently high securing force. In addition, a titanium alloy has various characteristics different from pure titanium. For example, Ti-6Al-4V has a light weight, high corrosion resistance, high strength, and high fatigue strength. The titanium-alloy implant body 2 according to the present embodiment including a titanium alloy with distinct features can be implanted into and secured to the base with a high securing force equivalent to that of a pure-titanium implant body.

According to the invention, the first curve of the outer circumferential surface of the implant body 2 is not used. The waviness component is removed from the first curve by applying a Gaussian filter to the first curve at a cutoff value of 5 µm. The surface roughness value Ra or the arithmetic mean roughness value for a second curve obtained after removing the waviness component is used. More specifically, the interrelation (correlation) between the removal torque value and the surface roughness value Ra is obtained with the effect of the waviness component being excluded. This causes a drastic change in the removal torque value at the surface roughness value Ra of 0.2 µm as shown in FIG. 3. Thus, the technique according to the present embodiment differs from a known technique of using the interrelation between the securing force and the surface roughness value of the implant body 2 containing the waviness component.

For an example relationship between the etching conditions and the surface roughness value Ra obtained after removing the waviness component, the surface roughness values Ra were 0.29, 0.30, and 0.24 µm respectively for Ti-6Al-4V samples treated for 30 seconds, one minute, and two minutes with an etchant containing an ammonium fluoride, a sulfuric acid, and a hydrogen peroxide. The surface roughness value Ra was 0.10 µm before etching.

In the above torque test conducted on the implant body prepared under these conditions, the implant body showed a high securing force with the bone, or removal torque of greater than or equal to 0.40 Nm.

A Ti-6Al-7Nb alloy treated with an etchant containing an ammonium fluoride, a sulfuric acid, and a hydrogen peroxide for one minute had a surface roughness value Ra of 0.25 µm. A Ti-6Al-2Nb-1Ta alloy treated with the same etchant for one minute had a surface roughness value Ra of 0.26 µm.

In comparison with the above, a Ti-6Al-4V alloy treated with a known method with an etchant containing 1 wt% of a sodium fluoride and 0.25 N of a hydrochloric acid at 25 °C for five minutes had a surface roughness value Ra of 0.10 µm. A Ti-6Al-4V alloy treated with an etchant containing 0.1 wt% of a sodium fluoride and 0.25 N of a hydrochloric acid had a surface roughness value Ra of 0.04 µm. A Ti-6Al-4V alloy treated with an etchant containing 0.5 wt% of a sodium fluoride, 1 N of a hydrochloric acid, and 2 wt% of a sulfuric acid natrium had a surface roughness value Ra of 0.04 µm.

A Ti-6Al-4V alloy treated with another known method with an etchant containing a hydrogen fluoride and a nitric acid or with an etchant containing a hydrochloric acid and a sulfuric acid at 120 °C for five minutes had a surface roughness value Ra of 0.06 or 0.14 µm.

In the above example, fine unevenness is defined on the outer circumferential surface of the implant body 2 through etching using an etchant. Thus, with the implant body 2 having the outer circumferential surface with a porous structure, the etchant can enter the inside of the porous structure. Thus, the inner surface of the porous structure can uniformly have fine unevenness with a surface roughness value Ra of greater than or equal to 0.2 µm. Thus, fine unevenness can be defined throughout the outer circumferential surface of the implant body 2. The implant body 2 can thus be fully engaged with the base, and can be more firmly implanted into the base.

In the above example, blasting is performed as an example of macro surface roughening. Instead of or in addition to blasting, another process for forming a porous structure may be performed as macro surface roughening. For example, thermal spraying (or thermal spraying process) may be performed to form a porous structure on the outer circumferential surface of the implant body 2. In some embodiments, a porous structure may be formed on the outer circumferential surface of the implant body 2 by allowing at least one of a highly biocompatible metal particle, fiber, and wire to adhere to the outer circumferential surface of the implant body 2 in a dispersed manner.

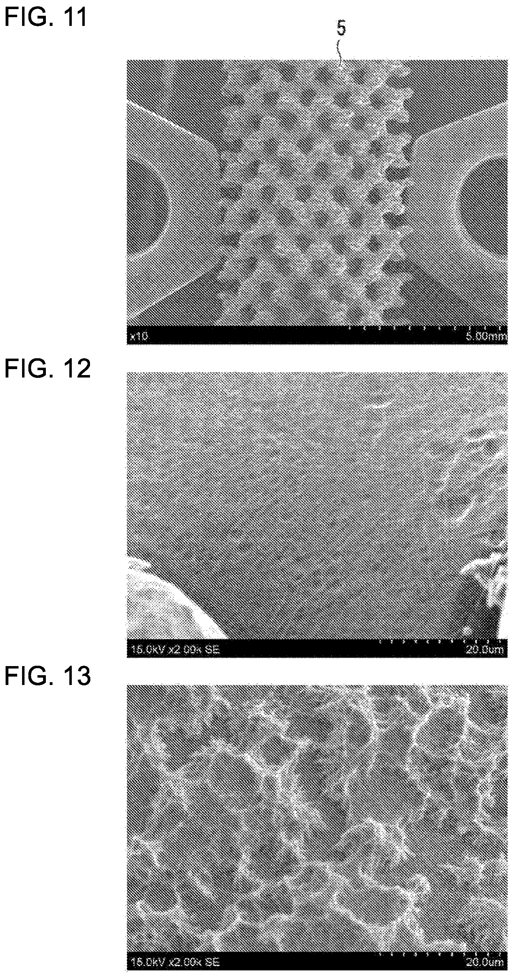

In some embodiments, for an implant such as an orthopedic implant, the porous structure may be formed on the outer circumferential surface of the implant body 2 through three-dimensional additive manufacturing using, for example, electron beams or a laser. Three-dimensional additive manufacturing enables formation of a complex bone shape (e.g., the shape of the skull or an acetabular replacement portion for the artificial hip joint), and formation of the porous structure on its surface. Three-dimensional additive manufacturing is also applicable to, for example, a spine cage, a spine device, or an artificial knee joint. FIG. 11 is a captured image of a cylindrical Ti-6Al-4V porous member 5 with a diameter of about 5 mm and a length of about 10 mm prepared through three-dimensional additive manufacturing. The porous member 5 in FIG. 11 has pores with a pore diameter of about 640 µm at a porosity of 64%. Three-dimensional additive manufacturing can form the implant body 2 including the porous member 5 integrally. A step of forming a porous structure through three-dimensional additive manufacturing corresponds to a step including preparation in step S1 and macro surface roughening in step S2.

Acid etching was performed on the porous member 5 with an etchant containing an ammonium fluoride, a sulfuric acid, and a hydrogen peroxide. The inner surface of the porous member 5 also had a surface roughness value of a sub-micrometer order equivalent to the surface roughness value obtained through etching performed on the blasted surface treated through blasting. FIG. 12 is a high-power-field SEM photo of the pore inner surface of the porous member 5 captured with a magnification of 5000 before etching. The pore inner surface of the porous member 5 had a surface roughness value Ra of 0.08 µm measured by the above measurement method excluding the waviness component. In contrast, FIG. 13 is a SEM photo (with a magnification of 5000) of the pore inner surface of the porous member 5 after etching. The pore inner surface had a surface roughness value Ra of 0.35 µm. Thus, the etching with an etchant allows a porous member having a complex shape to have a surface roughness value Ra of greater than or equal to 0.2 µm.

The upper limit of the range of the surface roughness value Ra is, for example, 0.6 µm. In other words, the surface roughness value Ra may be specifically in a range greater than or equal to 0.2 µm and less than or equal to 0.6 µm. The implant body 2 having a surface roughness value Ra within this range can be firmly implanted into the base while achieving a sufficiently high removal torque value.

Sufficient engagement between the implant body 2 and the base may be achieved based also on the cycle of the unevenness on the outer circumferential surface of the implant body 2. The cycle of the unevenness may be shorter. In other words, the spatial frequency of the unevenness may be higher.

As an index including the cycle of the unevenness on the outer circumferential surface of the implant body 2, a surface roughness value Rlr described below is used. The surface roughness value Rlr is the ratio of the length of the first curve or the profile curve of the implant body 2 to the measurement length. The length of the first curve is the length of the first curve imaginary straightened into a straight line. As the cycle of the unevenness on the outer circumferential surface of the implant body 2 is shorter, the first curve is longer, and the surface roughness value Rlr is higher. In other words, for the surface roughness value Ra being fixed, the cycle of the unevenness is shorter as the surface roughness value Rlr is higher.

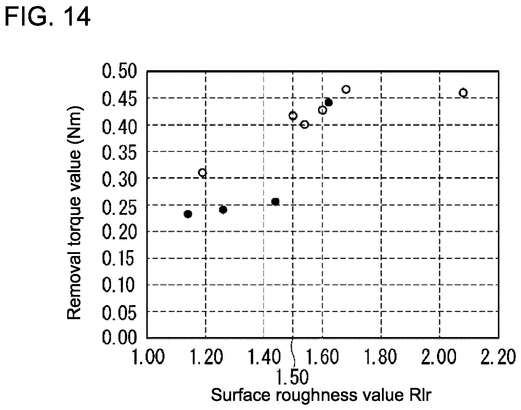

FIG. 14 is a graph showing an example of the relationship between the surface roughness value Rlr and the removal torque value. The graph in FIG. 14 is obtained using the above ten implant bodies. The surface roughness value Rlr is calculated through image processing on the captured image. More specifically, as described above, the image processing device first obtains the first curve based on the captured image. The image processing device then calculates the length of the first curve. For example, the image processing device sequentially calculates the distance between every adjacent two of the pixels indicating the first curve in the captured image, and the sum of the distances is obtained as the length of the first curve. The image processing device then divides the length of the first curve with the measurement length (= 25.4 µm) to calculate the surface roughness value Rlr (= first curve length/measurement length). The image processing device calculates the surface roughness value Rlr for each implant body.

The graph in FIG. 14 was obtained by plotting the surface roughness value Rlr of each measured implant body and the removal torque value of the implant body. In the example in FIG. 14, data pieces for the pure-titanium implant bodies are indicated with plotted points of outlined circles, and data pieces for the titanium-alloy implant bodies 2 are indicated with plotted points of solid circles.

As seen from FIG. 14, when the surface roughness value Rlr is below 1.5, the removal torque value is less than or equal to a value about slightly greater than 0.3 Nm, whereas when the surface roughness value Rlr is greater than or equal to 1.5, the removal torque value is greater than or equal to about 0.4 Nm. As seen from FIG. 14, the removal torque value changes drastically at the surface roughness value Rlr of about 1.5.

Thus, the implant body 2 may have the surface roughness value Rlr of greater than or equal to 1.5. The implant body 2 with this structure can be more firmly secured to the bone.

As described above, although the implant 1 and the method for manufacturing the implant 1 have been described in detail, the above structure and method are illustrative in all respects, and the disclosure is not limited to the above structure and method. The variations described above may be combined unless any contradiction arises. Many variations not specifically described above may be implemented without departing from the scope of the disclosure.

For example, instead of or in addition to etching, laser processing may be performed. The implant body 2 may thus have a surface roughness value Ra of greater than or equal to 0.2 µm.

### REFERENCE SIGNS

- 1: implant
- 2: implant body

## Claims

1. A biological implant (1) implantable in a base being a part of a living organism, the biological implant (1) comprising:
an implant body (2) implantable in the base,
**characterized by**
the implant body (2) including at least an outer circumferential surface comprising a titanium alloy, and
based on a first curve indicating a profile of the outer circumferential surface of the implant body (2) obtained with a measurement length of 25.4 µm and a second curve obtained by removing a long-wavelength component from the first curve by applying a Gaussian filter to the first curve at a cutoff value of 5 µm in image processing performed on an image of a cut surface of the implant body (2) perpendicular to the outer circumferential surface of the implant body (2), an arithmetic mean roughness value of the outer circumferential surface calculated using the second curve is greater than or equal to 0.2 µm.

2. The biological implant (1) according to claim 1, wherein
the titanium alloy includes an α-β alloy.

3. The biological implant (1) according to claim 2, wherein
the titanium alloy includes a titanium-6 aluminum-4 vanadium alloy.

4. The biological implant (1) according to claim 2, wherein
the titanium alloy includes a titanium-6 aluminum-7 niobium alloy or a titanium-6 aluminum-2 niobium-1 tantalum alloy.

5. The biological implant (1) according to any one of claims 1 to 4, wherein
the arithmetic mean roughness value is less than or equal to 0.6 µm.

6. The biological implant (1) according to any one of claims 1 to 5, wherein
the outer circumferential surface of the implant body (2) includes a porous structure, and the porous structure has an inner surface with the arithmetic mean roughness value of greater than or equal to 0.2 µm.

7. The biological implant (1) according to any one of claims 1 to 6, wherein
the biological implant (1) includes a dental implant, a spinal implant, or an implant for joint replacement.

8. A method for manufacturing the biological implant (1) according to any one of claims 1 to 7, the method comprising:
preparing an untreated implant body including at least an outer circumferential surface comprising a titanium alloy; and
etching the outer circumferential surface of a semi-treated implant body with an acid mixture containing at least an ammonium fluoride, a sulfuric acid, and a hydrogen peroxide solution.

9. The method according to claim 8, wherein
the etching the outer circumferential surface of the semi-treated implant body includes
subjecting the untreated implant body to macro surface roughening to obtain the semi-treated implant body, and
performing the etching on the semi-treated implant body.

10. The method according to claim 9, wherein
the macro surface roughening includes blasting.

11. The method according to claim 9 or claim 10, wherein
the semi-treated implant body has a surface roughness value of greater than or equal to 1 µm and less than or equal to 3 µm.

12. The method according to any one of claims 9 to 11, wherein
the macro surface roughening includes thermal spraying.

13. The method according to any one of claims 8 to 12, wherein
the etching is performed for a period longer than or equal to ten seconds and shorter than or equal to ten minutes.

14. The method according to any one of claims 8 to 13, wherein
the preparing the untreated implant body includes forming a porous structure through three-dimensional additive manufacturing.

## Patentansprüche

1. Biologisches Implantat (1), das in eine Basis implantiert werden kann, die ein Teil eines lebenden Organismus ist, wobei das biologische Implantat (1) umfasst:
einen Implantatkörper (2), der in die Basis implantiert werden kann,
**dadurch gekennzeichnet, dass**
der Implantatkörper (2) mindestens eine äußere Umfangsfläche mit einer Titanlegierung aufweist, und
auf der Grundlage einer ersten Kurve, die ein Profil der äußeren Umfangsfläche des Implantatkörpers (2) angibt, die mit einer Messlänge von 25,4 µm erhalten wird, und einer zweiten Kurve, die erhalten wird durch Entfernen einer langwelligen Komponente aus der ersten Kurve durch Anwenden eines Gauß-Filters auf die erste Kurve bei einem Cutoff-Wert von 5 µm in einer Bildverarbeitung, die an einem Bild einer Schnittfläche des Implantatkörpers (2) senkrecht zur äußeren Umfangsfläche des Implantatkörpers (2) durchgeführt wird, ein arithmetischer mittlerer Rauheitswert der äußeren Umfangsfläche, der unter Verwendung der zweiten Kurve berechnet wird, größer als oder gleich 0,2 µm ist.

2. Biologisches Implantat (1) gemäß Anspruch 1, wobei
die Titanlegierung eine α-β-Legierung umfasst.

3. Biologisches Implantat (1) gemäß Anspruch 2, wobei
die Titanlegierung eine Titan-6-Aluminium-4-Vanadium-Legierung umfasst.

4. Biologisches Implantat (1) gemäß Anspruch 2, wobei
die Titanlegierung eine Titan-6-Aluminium-7-Niob-Legierung oder eine Titan-6-Aluminium-2-Niob-1-Tantal-Legierung aufweist.

5. Biologisches Implantat (1) gemäß einem der Ansprüche 1 bis 4, wobei
der arithmetische mittlere Rauheitswert kleiner als oder gleich 0,6 µm ist.

6. Biologisches Implantat (1) gemäß einem der Ansprüche 1 bis 5, wobei
die äußere Umfangsfläche des Implantatkörpers (2) eine poröse Struktur aufweist und die poröse Struktur eine innere Oberfläche mit einem arithmetischen mittleren Rauheitswert von mehr als oder gleich 0,2 µm hat.

7. Biologisches Implantat (1) gemäß einem der Ansprüche 1 bis 6, wobei
das biologische Implantat (1) ein Zahnimplantat, ein Wirbelsäulenimplantat oder ein Implantat zum Gelenkersatz umfasst.

8. Verfahren zur Herstellung des biologischen Implantats (1) nach einem der Ansprüche 1 bis 7, wobei das Verfahren umfasst:
Herstellen eines unbehandelten Implantatkörpers mit mindestens einer äußeren Umfangsfläche mit einer Titanlegierung; und
Ätzen der äußeren Umfangsfläche eines halbbehandelten Implantatkörpers mit einem Säuregemisch, das mindestens ein Ammoniumfluorid, eine Schwefelsäure und eine Wasserstoffperoxidlösung enthält.

9. Verfahren gemäß Anspruch 8, wobei
das Ätzen der äußeren Umfangsfläche des halbbehandelten Implantatkörpers umfasst
Unterziehen des unbehandelten Implantatkörpers einer Makro-Oberflächenaufrauhung, um den halbbehandelten Implantatkörper zu erhalten, und
Durchführen der Ätzung auf dem halbbehandelten Implantatkörper.

10. Verfahren gemäß Anspruch 9, wobei
das Aufrauen der Makrooberfläche Sandstrahlen umfasst.

11. Verfahren nach Anspruch 9 oder Anspruch 10, wobei
der halbbehandelte Implantatkörper einen Oberflächenrauheitswert von größer als oder gleich 1 µm und kleiner als oder gleich 3 µm aufweist.

12. Verfahren gemäß einem der Ansprüche 9 bis 11, wobei
die Makro-Oberflächenaufrauung thermisches Spritzen umfasst.

13. Verfahren gemäß einem der Ansprüche 8 bis 12, wobei
das Ätzen für eine Dauer durchgeführt wird, die länger als oder gleich zehn Sekunden und kürzer als oder gleich zehn Minuten ist.

14. Verfahren gemäß einem der Ansprüche 8 bis 13, wobei
die Herstellung des unbehandelten Implantatkörpers die Bildung einer porösen Struktur durch dreidimensionale additive Fertigung umfasst.

## Revendications

1. Implant biologique (1) implantable dans une base faisant partie d'un organisme vivant, l'implant biologique (1) comprenant :
un corps d'implant (2) implantable dans la base,
**caractérisé en ce que**
le corps d'implant (2) comprend au moins une surface circonférentielle extérieure comprenant un alliage de titane, et
sur la base d'une première courbe indiquant un profil de la surface circonférentielle extérieure du corps d'implant (2) obtenue avec une longueur de mesure de 25,4 µm et d'une seconde courbe obtenue en éliminant une composante de grande longueur d'onde de la première courbe en appliquant un filtre gaussien à la première courbe à une valeur de coupure de 5 µm dans le traitement d'image effectué sur une image d'une surface coupée du corps d'implant (2) perpendiculaire à la surface circonférentielle extérieure du corps d'implant (2), une valeur de rugosité moyenne arithmétique de la surface circonférentielle extérieure calculée à l'aide de la seconde courbe est supérieure ou égale à 0,2 µm.

2. Implant biologique (1) selon la revendication 1, dans lequel
l'alliage de titane comprend un alliage α-β.

3. Implant biologique (1) selon la revendication 2, dans lequel
l'alliage de titane comprend un alliage titane-6 aluminium-4 vanadium.

4. Implant biologique (1) selon la revendication 2, dans lequel
l'alliage de titane comprend un alliage titane-6 aluminium-7 niobium ou un alliage titane-6 aluminium-2 niobium-1 tantale.

5. Implant biologique (1) selon l'une quelconque des revendications 1 à 4, dans lequel
la valeur de rugosité moyenne arithmétique est inférieure ou égale à 0,6 µm.

6. Implant biologique (1) selon l'une quelconque des revendications 1 à 5, dans lequel
la surface circonférentielle extérieure du corps d'implant (2) comprend une structure poreuse, et la structure poreuse présente une surface intérieure dont la valeur de rugosité moyenne arithmétique est supérieure ou égale à 0,2 µm.

7. Implant biologique (1) selon l'une quelconque des revendications 1 à 6, dans lequel
l'implant biologique (1) comprend un implant dentaire, un implant vertébral ou un implant pour le remplacement d'une articulation.

8. Procédé de fabrication de l'implant biologique (1) selon l'une quelconque des revendications 1 à 7, le procédé comprenant :
préparer un corps d'implant non-traité comprenant au moins une surface circonférentielle extérieure comprenant un alliage de titane ; et
graver la surface circonférentielle extérieure d'un corps d'implant semi-traité avec un mélange d'acides contenant au moins une solution de fluorure d'ammonium, d'acide sulfurique et de peroxyde d'hydrogène.

9. Procédé selon la revendication 8, dans lequel
la gravure de la surface circonférentielle extérieure du corps d'implant semi-traité comprend
soumettre le corps d'implant non-traité à une rugosification macroscopique de surface pour obtenir le corps d'implant semi-traité, et
effectuer la gravure sur le corps d'implant semi-traité.

10. Procédé selon la revendication 9, dans lequel
la rugosification macroscopique de surface comprend le sablage.

11. Procédé selon la revendication 9 ou revendication 10, dans lequel
le corps d'implant semi-traité présente une valeur de rugosité de surface supérieure ou égale à 1 µm et inférieure ou égale à 3 µm.

12. Procédé selon l'une quelconque des revendications 9 à 11, dans lequel
la rugosification macroscopique de surface comprend la pulvérisation thermique.

13. Procédé selon l'une quelconque des revendications 8 à 12, dans lequel
la gravure est effectuée pendant une période supérieure ou égale à dix secondes et inférieure ou égale à dix minutes.

14. Procédé selon l'une quelconque des revendications 8 à 13, dans lequel
la préparation du corps d'implant non-traité comprend la formation d'une structure poreuse par fabrication additive tridimensionnelle.
